# EUROPEAN PATENT APPLICATION

(11) **EP 4 141 447 A1**
(43) Date of publication of application: **01.03.2023**
(21) Application number: 21792790.4
(22) Date of filing: 26.03.2021
(51) Int. Cl.: G01N 33/545, G01N 21/64, G01N 33/543

(54) **DETECTION METHOD, DETECTION DEVICE, AND DIELECTRIC PARTICLES**

(30) Priority: 24.04.2020 JP 2020077016
(71) Applicant: Panasonic Intellectual Property Management Co., Ltd., Osaka-shi, Osaka 540-6207 (JP)
(72) Inventor: KANNO Takashi, Chuo-ku, Osaka-shi, Osaka 540-6207 (JP); HIRAOKA Rui, Chuo-ku, Osaka-shi, Osaka 540-6207 (JP); ARIMOTO Satoshi, Chuo-ku, Osaka-shi, Osaka 540-6207 (JP)
(74) Representative: Eisenführ Speiser
(86) International application number: PCT/JP2021/013077
(87) International publication number: WO 2021/215194

(57) **Abstract**

A detection method includes forming a complex by binding a target substance (11) and a dielectric particle (12a) modified by a single-domain antibody that is bindable to the target substance (11), separating the complex and an unbound particle (12) in a fluid with dielectrophoresis, the unbound particle being the dielectric particle (12a) not forming the complex, and detecting the target substance (11) contained in the separated complex with an imaging element (140).

## Description

### Technical Field

The present disclosure relates to a detection method and a detection device for detecting a target substance, such as a virus, and relates to a dielectric particle used in the detection method and the detection device.

### Background Art

An optical detection method of detecting a minute target substance with high sensitivity with the aid of a near field has been proposed so far. According to Patent Literature (PTL) 1, for example, a bound complex is formed by binding a target substance to a magnetic particle and a fluorescent particle, and the target substance is detected by measuring, for example, a fall of an optical signal, the fall being caused by application of a first magnetic field that causes the bound complex to move in a direction away from the surface of a detection plate where a near field is formed.

### Citation List

### Patent Literature

PTL 1: International Publication No. 2017/187744

With PTL 1, however, because a bound complex formed by nonspecific adsorption causing the magnetic particle and the fluorescent particle to bind to each other without the target substance interposed therebetween is also caused to move while emitting fluorescence, it is difficult to discriminate the bound complex not including the target substance from the bound complex including the target substance. This leads to false-positive detection that the target substance is falsely detected with the bound complex not including the target substance. Hence detection accuracy is reduced.

In view of the above-described problem, the present disclosure provides a target substance detection method and so on capable of reducing a possibility of the false-positive detection caused by the nonspecific adsorption and improving the detection accuracy of the target substance.

A detection method according to one aspect of the present disclosure includes forming a complex by binding a target substance and a dielectric particle modified by a single-domain antibody that is bindable to the target substance, separating the complex and an unbound particle in a fluid with dielectrophoresis, the unbound particle being the dielectric particle not forming the complex, and detecting the target substance contained in the separated complex with an imaging element.

A detection device according to one aspect of the present disclosure detects a target substance with dielectrophoresis, the detection device including a dielectric particle modified by a single-domain antibody that is bindable to the target substance. a separator separating a complex formed by binding of the target substance and the dielectric particle from an unbound particle in a fluid with the dielectrophoresis, the unbound particle being the dielectric particle not forming the complex, and an imaging element used to detect the target substance contained in the separated complex.

A dielectric particle according to one aspect of the present disclosure is a dielectric particle modified by a single-domain antibody that is bindable to a target substance.

General or specific embodiments of the present disclosure may be implemented not only in forms of a system, an integrated circuit, a computer program, or a computer-readable recording medium, but also in any selective combinations of a method, a device, a system, an integrated circuit, a computer program, and a computer-readable recording medium. The computer-readable recording medium includes, for example, a nonvolatile recording medium such as a CD-ROM (Compact Disc - Read Only Memory).

The detection method and so on according to the aspects of the present disclosure can reduce the false-positive detection caused by the nonspecific adsorption and can improve the detection accuracy of the target substance.

### Brief Description of Drawings

[Fig. 1] Fig. 1 is a perspective view illustrating a schematic configuration of a detection device according to an embodiment.
[Fig. 2] Fig. 2 is a sectional view illustrating a schematic configuration of the detection device according to the embodiment.
[Fig. 3] Fig. 3 is a plan view illustrating a configuration of an electrode set in the embodiment.
[Fig. 4] Fig. 4 is a flowchart illustrating a detection method according to an embodiment.
[Fig. 5] Fig. 5 illustrates a formation process of a complex particle in the embodiment.
[Fig. 6] Fig. 6 is a graph depicting a set frequency of an alternate current voltage in the embodiment.
[Fig. 7A] Fig. 7A is a schematic view illustrating particles having moved to a first electric field zone.
[Fig. 7B] Fig. 7B is a schematic view illustrating the particles having moved to the first electric field zone and a second electric field zone.
[Fig. 7C] Fig. 7C is a schematic view illustrating the particles having moved to the second electric field zone.
[Fig. 8] Fig. 8 is a graph depicting a crossover frequency for each particle type in the embodiment.
[Fig. 9A] Fig. 9A is a first graph depicting zeta potentials of particles in the embodiment and a comparative example.
[Fig. 9B] Fig. 9B is a second graph depicting the zeta potentials of the particles in the embodiment and the comparative example.
[Fig. 10] Fig. 10 is a first plan view illustrating a configuration of an electrode set according to a modification.
[Fig. 11] Fig. 11 is a second plan view illustrating a configuration of an electrode set according to a modification.
[Fig. 12] Fig. 12 illustrates a formation process of a complex particle according to a modification.

### Description of Embodiments

Embodiments will be described below with reference to the drawings.

It is to be noted that each of the following embodiments represents a general or specific example. Numerical values, shapes, materials, constituent elements, layout positions and connection forms of the constituent elements, steps, order of the steps, etc., which are described in the following embodiments, are merely illustrative, and they are not purported to limit the scope of Claims. The drawings are not always exactly drawn in a strict sense. In the drawings, substantially the same constituent elements are denoted by the same reference sings, and duplicate description of those constituent elements is omitted or simplified in some cases.

In the following description, terms representing relationships between the constituent elements, such as "parallel" and "vertical", terms representing shapes of the constituent elements, such as "rectangular", and a numerical range are to be interpreted as not exactly expressing the meaning of the terms and the range in a strict sense but as including substantially equivalent meaning and range with an allowance of serval %, for example.

In the following description, the wording "detect a target substance" indicates not only a process of finding the target substance and checking the presence of the target substance, but also a process of measuring an amount (for example, the number or concentration) of the target substances or a range of the amount.

### (Embodiments)

In the embodiments described below, a complex particle and an unbound particle are separated from each other in a fluid with DEP (dielectrophoresis), and a target substance contained in the separated complex particle is detected.

The term "dielectrophoresis" indicates a phenomenon that a force acts on a dielectric particle exposed to a nonuniform electric field. This force is developed without charging of the particle.

The term "target substance" indicates a substance to be detected and is, for example, a molecule such as a pathogenic protein, a virus (such as a capsid protein), or a bacterium (such as a polysaccharide). The target substance is also called a substance to be detected or a detection target in some cases.

Examples of a detection device and a detection method implementing the detection of the target substance with the dielectrophoresis will be described in detail below with reference to the drawings.

### [Configuration of Detection Device]

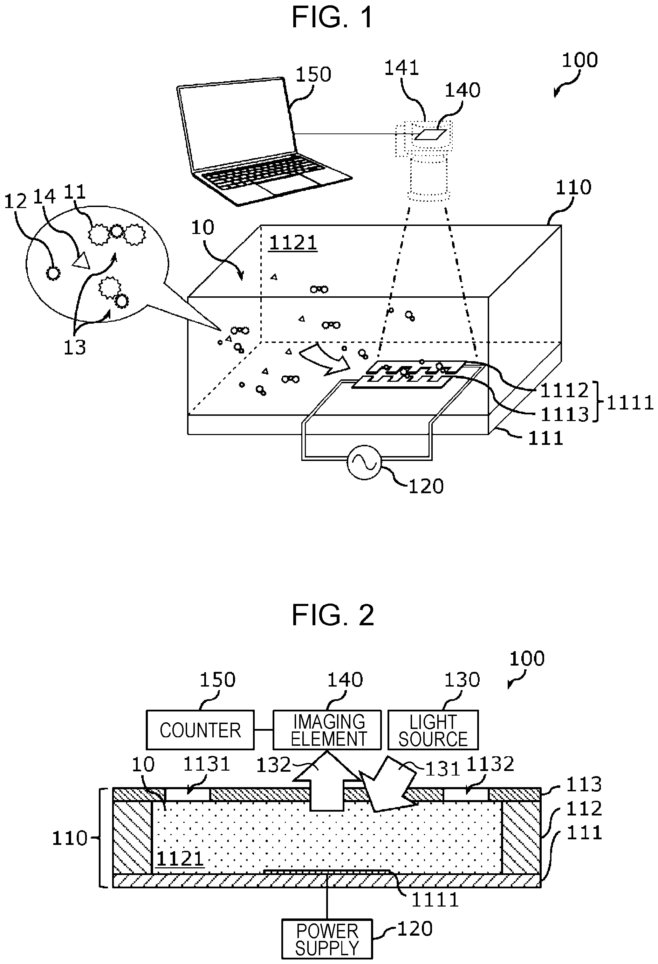

First, a configuration of the detection device is described with reference to Figs. 1 and 2. Fig. 1 is a perspective view illustrating the schematic configuration of the detection device according to the embodiment. Fig. 2 is a sectional view illustrating a schematic configuration of the detection device according to the embodiment. More specifically, in Fig. 1, an outline of a separator 110 is illustrated in a fashion seeing through portions other than a first substrate 111 such that the inside of the separator 110 is recognized. Fig. 1 is to explain relationships between the separator 110 as a main component and other components and is not purported to limit layout positions, layout directions, postures, and so on of the individual components when the detection device 100 is used. Fig. 2 is a sectional view of the separator 110, illustrated in Fig. 1, cut along a direction parallel to the drawing sheet. Thicknesses of some components of the separator 110 illustrated in Fig. 2 are not illustrated in Fig. 1.

As illustrated in Figs. 1 and 2, the detection device 100 includes the separator 110, a power supply 120, a light source 130, an imaging element 140, and a detector 150.

The separator 110 is a vessel for storing a sample 10 containing the target substance 11 and includes a space 1121 therein. The sample 10 is stored in the space 1121. In the separator 110, a complex particle 13 and an unbound particle 12 are separated from each other in a fluid (i.e., a solvent fluid of the sample 10) inside the space 1121 with the dielectrophoresis. In the embodiment, the separator 110 separates the complex particle 13 and the unbound particle 12 in terms of position. The sample 10 produced after mixing of the target substance 11 includes the complex particle 13 made up of the target substance 11 and a dielectric particle 12a bound to each other, and the unbound particle 12. A contaminant 14 is mixed into the sample 10 in some cases.

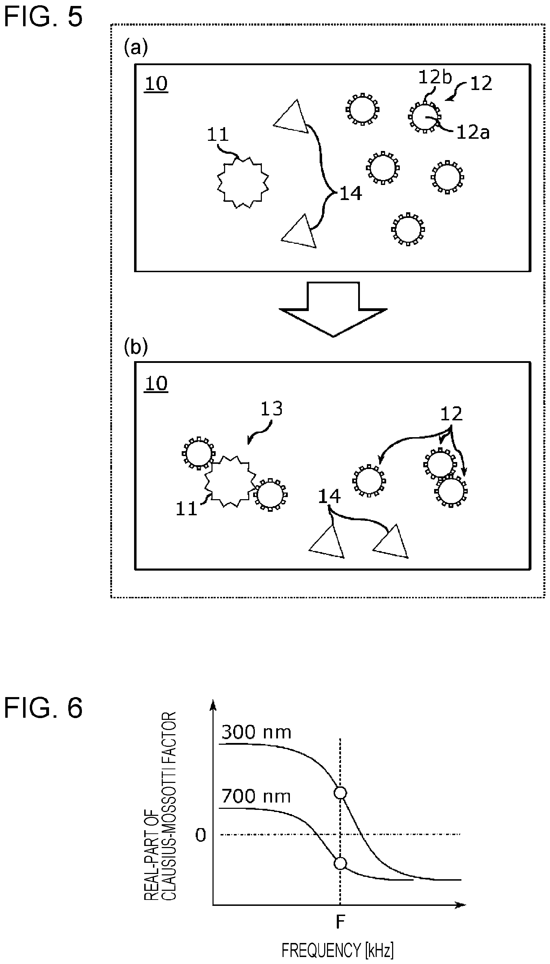

The complex particle 13 is a complex produced by binding of the target substance 11 and the dielectric particle 12a (see Fig. 5 described later) that is modified by a substance with the properties of specifically binding to the target substance 11. In other words, in the complex particle 13, the target substance 11 and the dielectric particle 12a are bound to each other in a state in which the substance with the properties of specifically binding to the target substance 11 is interposed therebetween.

The dielectric particle 12a is a particle capable of being polarized with an electric field applied. The dielectric particle 12a may contain, for example, a fluorescent substance. When the dielectric particle 12a is illuminated with light emitted from a light source 130 (described later) and having a wavelength at which the fluorescent substance is excited, the dielectric particle 12a can be detected by detecting light in a wavelength band of fluorescence emission. The dielectric particle 12a is not limited to a particle containing the fluorescent substance. For example, a polystyrene particle or a glass particle each not containing the fluorescent substance may be used as the dielectric particle 12a.

The substance with the properties of specifically binding to the target substance is a substance capable of specifically binding to the target substance 11 and is also called a specifically binding substance 12b (see Fig. 5 described later). A single-domain antibody, particularly a VHH antibody, for a target substance is used as the specifically binding substance 12b for the target substance 11.

The unbound particle 12 includes the dielectric particle 12a not forming the complex particle 13. Thus, the unbound particle 12 is the dielectric particle 12a that is modified by the specifically binding substance 12b and that is not bound to the target substance 11. The unbound particle 12 is also called a free (F) component. On the other hand, the complex particle 13 contains the dielectric particle 12a that is bound to the target substance 11 and that is modified by the specifically binding substance 12b. The dielectric particle 12a and the specifically binding substance 12b being bindable to the target substance 11 are each also called a binding (B) component.

An inner configuration of the separator 110 is described here. As illustrated in Fig. 2, the separator 110 includes the first substrate 111, a spacer 112, and a second substrate 113.

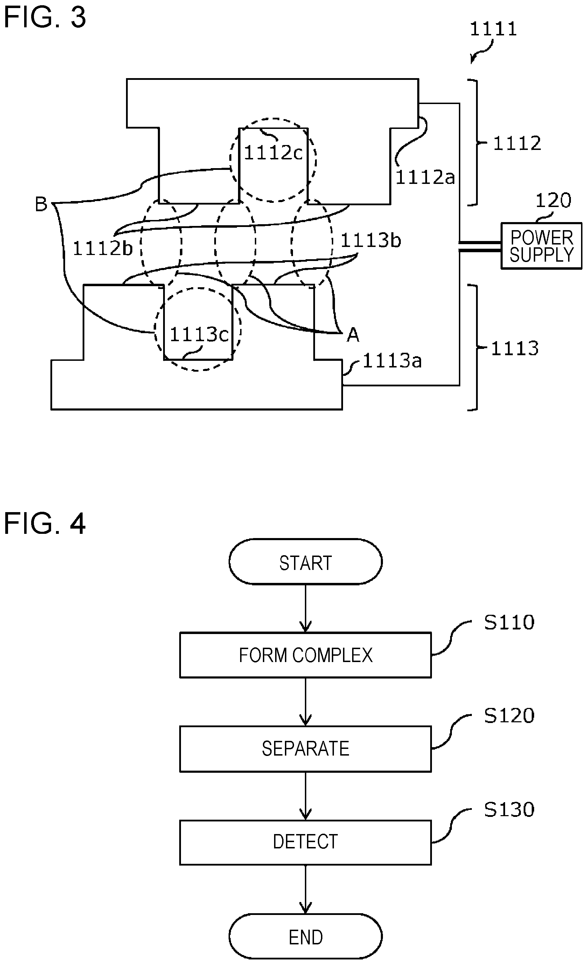

The first substrate 111 is a sheet made of glass or resin, for example. The first substrate 111 has an upper surface defining the bottom of the space 1121, and an electrode set 1111 to which an alternate current voltage is applied from the power supply 120 is formed on the upper surface. The electrode set 1111 includes a first electrode 1112 and a second electrode 1113 and can generate a nonuniform electric field (also called an electric field gradient) on the first substrate 111. Thus, the electrode set 1111 is an example of an electric field gradient generator generating (or forming) the electric field gradient. Details of the electrode set 1111 will be described later with reference to Fig. 3.

The spacer 112 is disposed on the first substrate 111. A through-hole corresponding to a shape of the space 1121 is formed in the spacer 112. In other words, the space 1121 is formed by the through-hole sandwiched between the first substrate 111 and the second substrate 113. As described above, the sample 10 containing the complex particle 13 and the unbound particle 12 is introduced to the space 1121. The spacer 112 is an outer wall surrounding the through-hole and has an inner surface defining the space 1121. The spacer 112 is made of, for example, a material, such as resin, with high adhesion to the first substrate 111 and the second substrate 113.

The second substrate 113 is a transparent sheet made of glass or resin, for example, and is disposed on the spacer 112. For example, a polycarbonate substrate can be used as the second substrate 113. A supply hole 1131 and a discharge hole 1132 each in communication with the space 1121 are formed in the second substrate 113 to penetrate therethrough. The sample 10 is supplied to the space 1121 through the supply hole 1131 and is discharged from the space 1121 through the discharge hole 1132. The separator 110 may be constituted without including the second substrate 113. Thus, the second substrate 113 is not an essential constituent element. The space 1121 with which the separator 110 is realized as the vessel is formed by the first substrate 111 and the spacer 112 that define respectively the bottom and the inner surface of the vessel.

The power supply 120 is an alternate current power supply and applies an alternate current voltage to the electrode set 1111 on the first substrate 111. The power supply 120 may be any type of power supply insofar as it can supply the alternate current voltage, and is not limited to a particular power supply. The alternate current voltage may be supplied from an external power supply. In that case, the power supply 120 does not need to be included in the detection device 100.

The light source 130 emits illumination light 131 to the sample 10 in the space 1121. The illumination light 131 illuminates the sample 10 after passing through the transparent second substrate 113. Detection light 132 corresponding to the illumination light 131 is generated from the sample 10, and the dielectric particle 12a contained in the sample 10 is detected by detecting the detection light 132. For instance, when the dielectric particle 12a contains the fluorescent substance as described above, the fluorescent substance is excited by illuminating the dielectric particle 12a with the illumination light 131 as excitation light, and fluorescence generated from the fluorescent substance is detected as the detection light 132.

A known suitable light source can be utilized as the light source 130 without any specific limitations. For example, a laser such as a semiconductor laser or a gas laser can be used as the light source 130. A wavelength at which an interaction with respect to an object in the target substance 11 is small is used as the wavelength of the illumination light 131 emitted from the light source 130. When the target substance 11 is a virus, for example, the illumination light 131 with the wavelength of longer than or equal to 325 nm and shorter than or equal to 2000 nm is selected. The wavelength of the illumination light 131 may be set to a wavelength (for example, longer than or equal to 600 nm and shorter than or equal to 850 nm) that can be obtained with the semiconductor laser.

The light source 130 does not need to be included in the detection device 100. For instance, when the size of the dielectric particle 12a is large, the dielectric particle 12a can be observed with a combination of optical elements such as lenses, and a light emission phenomenon such as fluorescence emission is no longer required. Thus, the dielectric particle 12a does not need to contain the fluorescent substance, and in this case the illumination light 131 does not need to be emitted from the light source 130. Solar light, a fluorescent lamp, or the like can be used instead of the light source 130, and the dielectric particle 12a can be detected by utilizing the incoming ambient light.

The imaging element 140 is, for example, a CMOS image sensor or a CCD image sensor. Upon receiving the detection light 132 generated from the sample 10, the imaging element 140 produces an image and outputs the image. For instance, the imaging element 140 is incorporated in a camera 141, for example, to be horizontally arranged parallel to the surface of the first substrate 111 and takes an image of a region corresponding to the electrode set 1111 through an optical element (not illustrated), such as a lens, included in the camera 141. Thus, the imaging element 140 is used to take an image of the complex particle 13 separated from the unbound particle 12 by the separator 110 and to detect the target substance 11 contained in the complex particle 13.

In an example in which the dielectric particle 12a contains the fluorescent substance, the imaging element 140 takes an image of the fluorescence generated from the fluorescent substance contained in the dielectric particle 12a. The detection device 100 may include a photodetector instead of the imaging element 140. In that case, the photodetector is only required to detect the detection light 132, such as the fluorescence, from a region on the first substrate 111 where the complex particles 13 after being separated with the dielectrophoresis are collected. When the photodetector is used instead of the imaging element 140 as mentioned above, an analysis and so on to be performed by the detector 150 (described later) is not needed. Thus, the detection device 100 can also be realized without including the detector 150.

In addition, the detection device 100 may include an optical lens and/or an optical filter between the light source 130 and the separator 110 and/or between the separator 110 and the imaging element 140. For example, a long-pass filter capable of blocking the illumination light 131 from the light source 130 and passing the detection light 132 therethrough may be disposed between the separator 110 and the imaging element 140.

The detector 150 obtains the image output from the imaging element 140 and detects the dielectric particle 12a contained in the sample 10 based on the obtained image. Particularly, the detection device 100 according to the embodiment can separately count the number of the complex particles 13 and the number of the unbound particles 12. In other words, the detector 150 can detect the dielectric particle 12a forming the complex particle 13 and the dielectric particle 12a contained in the unbound particle 12 while discriminating both the dielectric particles. Accordingly, the detector 150 can detect the complex particle 13 in the sample 10 by detecting the dielectric particle 12a based on the image.

In an example, the detector 150 uses a previously taken reference image not including the dielectric particle 12a and detects a bright spot with a different brightness value by comparing the obtained image and the reference image with each other. In more detail, when light emission is to be detected as the detection light 132, a spot with a higher brightness value in the obtained image than in the reference image may be detected as the bright spot. When transmitted light or scattered light, for example, is to be detected as the detection light 132, a spot with a lower brightness value in the obtained image than in the reference image may be detected as the bright spot. In such a manner, the detector 150 obtains a detection result of the complex particle 13 in the sample 10.

The detector 150 is realized by executing a program for the above-described image analysis with, for example, a circuit such as a processor and a storage unit such as a memory. Alternatively, the detector 150 may be realized with a dedicated circuit. The detector 150 is incorporated in a computer, for example.

### [Shape and Arrangement of Electrode Set on First Substrate]

A shape and an arrangement of the electrode set 1111 on the first substrate 111 will be described below with reference to Fig. 3. Fig. 3 is a plan view illustrating a configuration of the electrode set in the embodiment. Fig. 3 illustrates the configuration of the electrode set 1111 when viewed in plan from the side including the imaging element 140. For the sake of simplicity, Fig. 3 is given as a schematic view illustrating part of the electrode set 1111.

As described above, the electrode set 1111 includes the first electrode 1112 and the second electrode 1113 that are arranged on the first substrate 111. The first electrode 1112 and the second electrode 1113 are each electrically connected to the power supply 120.

The first electrode 1112 includes a first base portion 1112a extending in a first direction (i.e., a left-right direction on the drawing sheet of Fig. 3) and two first protruding portions 1112b protruding from the first base portion 1112a in a second direction (i.e., an up-down direction on the drawing sheet of Fig. 3) intersecting the first direction. A first recessed portion 1112c is formed between the two first protruding portions 1112b. The two first protruding portions 1112b are arranged to face the second electrode 1113. In other words, the first electrode 1112 includes the first protruding portions 1112b protruding from the first base portion 1112a in a direction intersecting the first direction toward the second electrode 1113 in a convex form. The first protruding portion 1112b is positioned to face a second recessed portion 1113c of the second electrode 1113. Lengths of the two first protruding portions 1112b and the first recessed portion 1112c in the first direction and in the second direction are all, for example, about 5 micrometers. Sizes of the two first protruding portions 1112b and the first recessed portion 1112c are not limited to the above-mentioned value.

A shape and a size of the second electrode 1113 are substantially the same as those of the first electrode 1112. In more detail, the second electrode 1113 also includes a second base portion 1113a extending in the first direction (i.e., the left-right direction on the drawing sheet of Fig. 3) and two second protruding portions 1113b protruding from the second base portion 1113a in the second direction (i.e., the up-down direction on the drawing sheet of Fig. 3) intersecting the first direction. The second recessed portion 1113c is formed between the two second protruding portions 1113b. The two second protruding portions 1113b are arranged to face the first electrode 1112. In other words, the second electrode 1113 includes the second protruding portions 1113b protruding from the second base portion 1113a in a direction intersecting the first direction toward the first electrode 1112 in a convex form. The second protruding portion 1113b is positioned to face the first recessed portion 1112c of the first electrode 1112.

With an alternate current voltage applied between the first electrode 1112 and the second electrode 1113, a nonuniform electric field is generated on the first substrate 111. An alternate current voltage applied to the first electrode 1112 and an alternate current voltage applied to the second electrode 1113 may have voltage waveforms that are the same with a phase difference therebetween. The phase difference between the applied alternate current voltages may be, for example, 180 degrees.

A layout position of the electrode set 1111 is not limited to a region on the first substrate 111. The electrode set 1111 is just required to be arranged in the vicinity of the sample 10 within the space 1121. Here, the vicinity of the sample 10 indicates a range in which an electric field can be generated inside the sample 10 with the alternate current voltage applied to the electrode set 1111. In other words, the electrode set 1111 may be in direct contact with the sample 10 within the space 1121 or may form, from the outside of the space 1121, the electric field in a region where the sample 10 is positioned.

### [Distribution of Intensity of Electric Field on First Substrate]

A distribution of intensity of the nonuniform electric field generated on the first substrate 111 will be described below with reference to Fig. 3.

As illustrated in Fig. 3, a first electric field zone A where the intensity of the electric field is relatively high and a second electric field zone B where the intensity of the electric field is relatively low are formed on the first substrate 111 by the nonuniform electric field. The first electric field zone A is a zone with higher intensity of the electric field than in the second electric field zone B, the zone being positioned between the first protruding portion 1112b and the second protruding portion 1113b facing each other. More specifically, the first electric field zone A is formed at a position where ends of the first protruding portion 1112b and the second protruding portion 1113b face each other in the first direction.

The intensity of the electric field depends on a distance between electrodes generating electric fields. The intensity of the electric field reduces as the distance between the electrodes becomes longer and increases as the distance between the electrodes becomes shorter. The position where the ends of the first protruding portion 1112b and the second protruding portion 1113b face each other in the first direction is a position where the distance between the first electrode 1112 and the second electrode 1113 is shortest in the electrode set 1111 and where the intensity of the electric field is maximum. The first electric field zone A is a zone covering a predetermined range including the position where the distance between the first electrode 1112 and the second electrode 1113 is shortest.

The second electric field zone B is a zone with lower intensity of the electric field than in the first electric field zone A, the zone being formed within a region between the first protruding portion 1112b and the second recessed portion 1113c facing each other or between the first recessed portion 1112c and the second protruding portion 1113b facing each other. That region includes a position where the distance between the first electrode 1112 and the second electrode is longest, and in that region, the intensity of the electric field is lower at a position closer to the first recessed portion 1112c or the second recessed portion 1113c. The second electric field zone B includes the bottom of each of the first recessed portion 1112c or the second recessed portion 1113c where the intensity of the electric field is especially low.

### [Detection Method Using Detection Device]

A method of detecting the target substance by using the above-described detection device 100 will be described below with reference to Figs. 4 to 6. Fig. 4 is a flowchart illustrating the detection method according to the embodiment.

First, the complex particle 13 is formed by binding the target substance 11 and the dielectric particle 12a modified by the specifically binding substance 12b (S110). A formation process of the complex particle 13 is described here with reference to Fig. 5. Fig. 5 illustrates the formation process of the complex particle in the embodiment.

As illustrated in (a) of Fig. 5, the target substance 11 and the unbound particle 12 are mixed with each other, and the sample 10 is produced.

In the present disclosure, a single-domain antibody, particularly a VHH antibody, for a target substance is used as the specifically binding substance 12b for the target substance 11. An antibody is an example of the substance with the properties of specifically binding to the target substance 11. In the embodiment, in addition to the VHH antibody, another single-domain antibody capable of binding to the target substance 11 may also be used. The single-domain antibody includes, for example, the VHH antibody, a recombinant obtained with recombination and expression of a single-domain peptide chain in a variable region of immunoglobulin including an epitope recognition site by utilizing a host such as E. coli, and a single-domain antibody in a broad sense, namely a target recognition molecule having molecular weight of about 7 to 20 kDa and made of helix-loop-helix forming polypeptide. Such a target recognition molecule is known as the so-called micro-antibody. Sizes of the target substance 11, the dielectric particle 12a, and the VHH antibody are respectively about 100 nanometers, about 300 nanometers, and about 5 nanometers. In the present disclosure, an antibody other than the single-domain antibody, for example, an IgG antibody, is not used as the substance that is specifically bindable to the target substance.

When the sample 10 illustrated in Fig. 5(a) is left to stand in a fluid for a predetermined time under a predetermined temperature, the target substance 11 and the unbound particle 12 are bound to each other by the antigen-antibody reaction, and the complex particle 13 is formed as illustrated in (b) of Fig. 5. On that occasion, a size of the complex particle 13 is about 700 nanometers.

Generally, when the target substance 11 of which content is unknown is to be detected, the unbound particles 12 are used in an excessive amount to form the complex particles 13 for almost all the target substances 11. An amount of the complex particles 13 is correlated to an amount of the target substances 11. Therefore, the amount of the target substances 11 can be indirectly detected by detecting the amount of the complex particles 13. In the embodiment, since the unbound particles 12 are used in the excessive amount, the unbound particles 12 having not bound to the target substances 11 remain in an isolated or aggregated state as illustrated in Fig. 5(b).

Fig. 5(b) illustrates an example of a configuration of the complex particle 13, and the configuration of the complex particle 13 is not limited to the illustrated one. In another example, the number of the dielectric particles 12a contained in the complex particle 13 may be one or three or more. In still another example, the number of the target substances 11 contained in the complex particle 13 may be two or more.

Returning to the description of the flowchart of Fig. 4, the complex particle 13 and the unbound particle 12 are separated from each other in the fluid (i.e., the solvent fluid of the sample 10) with the dielectrophoresis (S120). In more detail, the alternate current voltage is applied to the electrode set 1111, and the nonuniform electric field is generated in the sample 10 on the first substrate 111. Accordingly, the dielectrophoresis acts on the complex particle 13 and the unbound particle 12, thus causing each of the complex particle 13 and the unbound particle 12 to move. The contaminant 14 is also separated in a similar manner. The separation to be developed here is to separate the detection target from the others. In other words, the complex particle 13 is to be separated from the unbound particle 12 and the contaminant 14. The unbound particle 12 and the contaminant 14 do not need to be separated from each other. The unbound particles 12 in the aggregated state are decomposed to the individual unbound particles 12 in the isolated state.

In consideration of the above point, the frequency of the alternate current voltage applied to the electrode set 1111 is set to a predetermined frequency. The application of the alternate current voltage of the predetermined frequency enables the dielectrophoresis to act on the complex particle 13 and both the unbound particle 12 and the contaminant 14 in different directions. For instance, when the predetermined frequency at which negative dielectrophoresis (nDEP) acts on the complex particle 13 and positive dielectrophoresis (pDEP) acts on both the unbound particle 12 and the contaminant 14 is set as the frequency of the alternate current voltage, the complex particle 13 is caused to move to the second electric field zone B where the intensity of the electric field is relatively low, and the unbound particle 12 and the contaminant 14 are caused to move to the first electric field zone A where the intensity of the electric field is relatively high. As a result, the complex particle 13 is separated from the unbound particle 12 and the contaminant 14 in terms of position.

Here, the predetermined frequency of the alternate current voltage is described with reference to Fig. 6. Fig. 6 is a graph depicting a set frequency of the alternate current voltage in the embodiment. In the graph of Fig. 5, the vertical axis represents the real-part of Clausius-Mossotti factor, and the horizontal axis represents the frequency of the alternate current voltage applied to the electrode set 1111.

When the real-part of Clausius-Mossotti factor is positive, the positive dielectrophoresis acts on a particle, and the particle is caused to move to a zone where the intensity of the electric field is higher. Conversely, when the real-part of Clausius-Mossotti factor is negative, the negative dielectrophoresis acts on a particle, and the particle is caused to move to a zone where the intensity of the electric field is lower.

As depicted in Fig. 6, the real-part of Clausius-Mossotti factor depends on the size of the particle and the frequency. At a frequency F, the real-part of Clausius-Mossotti factor is negative for a particle of 700 nanometers corresponding to the size of the complex particle 13, and the real-part of Clausius-Mossotti factor is positive for a particle of 300 nanometers corresponding to the size of the unbound particle 12. Accordingly, setting the frequency F as the predetermined frequency of the alternate current voltage enables the negative dielectrophoresis to act on the complex particle 13 and the positive dielectrophoresis to act on the unbound particle 12.

Returning to the description of the flowchart of Fig. 4, the target substance 11 contained in the separated complex particle 13 is finally detected (S130). In an example, the imaging element 140 takes an image of the second electric field zone B and outputs the image including the complex particle 13. The detector 150 executes an image analysis on the output image and detects the complex particle 13. Thus, the target substance 11 contained in the complex particle 13 is detected.

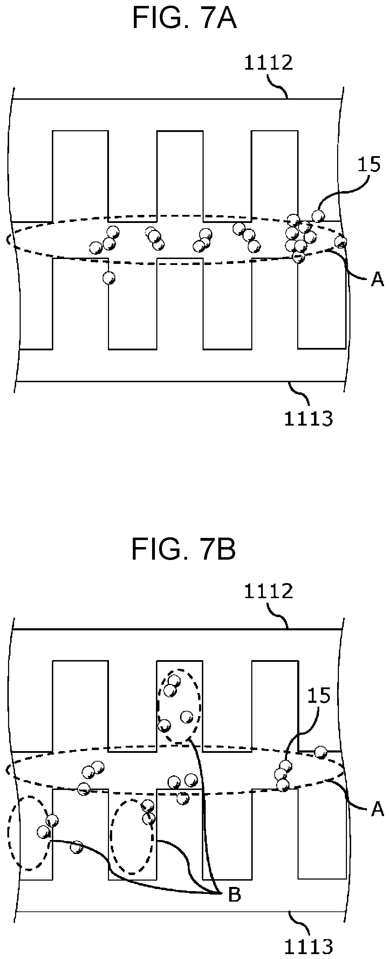

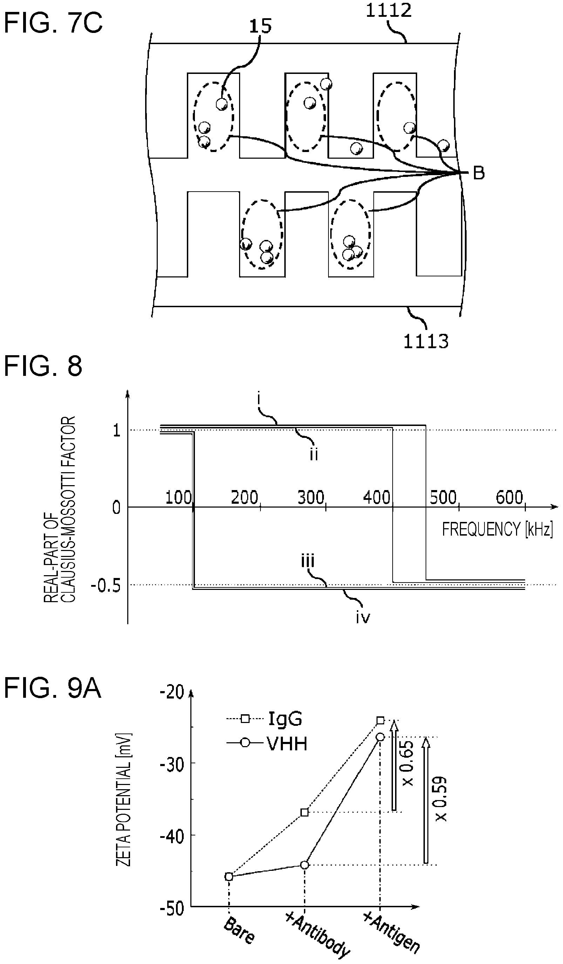

The setting of the above-described predetermined frequency will be described in more detail below with reference to Figs. 7A to 9B. Fig. 7A is a schematic view illustrating particles having moved to the first electric field zone. Fig. 7B is a schematic view illustrating the particles having moved to the first electric field zone and the second electric field zone. Fig. 7C is a schematic view illustrating the particles having moved to the second electric field zone. Figs. 7A to 7C are each a plan view illustrating the electrode set 1111 when viewed from the same direction as in Fig. 3, and particles 15 moving with the dielectrophoresis according to the electrode set 1111. The particles 15 illustrated here are general particles moving under the action of the dielectrophoresis and can be regarded as representing any of the complex particle 13, the unbound particle 12, and the contaminant 14.

As illustrated in Fig. 7A, when the alternate current voltage of the frequency at which the real-part of Clausius-Mossotti factor for the particles 15 has a positive value is applied to the electrode set 1111, the particles 15 are caused to move to the first electric field zone A with the positive dielectrophoresis.

As illustrated in Fig. 7B, when the alternate current voltage of the frequency at which the real-part of Clausius-Mossotti factor for the particles 15 is near 0 is applied to the electrode set 1111, the particles 15 are caused to move to the first electric field zone A and the second electric field zone B with the positive dielectrophoresis. Such a situation is produced with the individual particles 15 exhibiting slightly different properties, thus resulting in a state in which the particles 15 moving with the positive dielectrophoresis and the particles 15 moving with the negative dielectrophoresis are mixed.

As illustrated in Fig. 7C, when the alternate current voltage of the frequency at which the real-part of Clausius-Mossotti factor for the particles 15 has a negative value is applied to the electrode set 1111, the particles 15 are caused to move to the second electric field zone B with the negative dielectrophoresis. Thus, the direction of the dielectrophoresis acting on the particles 15 is reversed from the positive dielectrophoresis to the negative dielectrophoresis depending on the frequency of the alternate current voltage applied to the electrode set. The frequency of the alternate current voltage at which the direction of the dielectrophoresis is reversed (hereinafter also referred to as a "crossover frequency") is different depending on the type of the particles 15. When the state of Fig. 7B continues for a wide frequency band (namely, over a certain frequency band width), a minimum frequency providing the state of Fig. 7B is defined as the crossover frequency.

Fig. 8 is a graph depicting the crossover frequency for each particle type in the embodiment. In Fig. 8, the vertical axis represents positive and negative ranges of the real-part of Clausius-Mossotti factor, and the horizontal axis represents the frequency of the alternate current voltage applied to the electrode set 1111. To indicate whether the real-part of Clausius-Mossotti factor is positive or negative, the vertical axis of Fig. 8 denotes +1 or -1 that is obtained by dividing a value of the real-part of Clausius-Mossotti factor by an absolute value of that value. The graph of Fig. 8 represents results for (i) dielectric particle alone, (ii) dielectric particle modified by VHH antibody + target substance, (iii) solitary dielectric particle modified by the VHH antibody, and (iv) solitary dielectric particle modified by the IgG antibody.

As depicted in Fig. 8, it is understood for any type of the particles 15 that the crossover frequency is confirmed to be present within a frequency range of higher than or equal to 100 kHz to lower than or equal to 500 kHz, and that the direction of the dielectrophoresis is reversed from the positive dielectrophoresis to the negative dielectrophoresis as the frequency increases. The crossover frequency is different for each type of the particles 15.

Although not depicted in the graph, the crossover frequency of the dielectric particle modified by the IgG antibody + the target substance is not confirmed to be present within the frequency range of higher than or equal to 100 kHz to lower than or equal to 500 kHz, and the real-part of Clausius-Mossotti factor always takes a negative value. Thus, it is considered that the crossover frequency of the dielectric particle modified by the IgG antibody + the target substance is present within a frequency range lower than 100 kHz. As described above, however, the frequency of the alternate current voltage at which the direction of the dielectrophoresis is reversed spans over a certain band width in many cases.

To separate (iv) the solitary dielectric particle modified by the IgG antibody and the dielectric particle modified by the IgG antibody + the target substance from each other, the predetermined frequency needs to be set such that one of those dielectric particles is caused to move to the first electric field zone A with the positive dielectrophoresis and the other dielectric particle is caused to move to the second electric field zone B with the negative dielectrophoresis. However, because the crossover frequency of (iv) the solitary dielectric particle modified by the IgG antibody and the crossover frequency of the dielectric particle modified by the IgG antibody + the target substance substantially match with each other, the particles 15 given as both the dielectric particles exhibit the same behavior in the dielectrophoresis even when the alternate current voltage of any frequency is applied.

By contrast, since there is a difference of 300 kHz between the crossover frequency of (ii) the dielectric particle modified by the VHH antibody + target substance and the crossover frequency of (iii) the solitary dielectric particle modified by the VHH antibody, it is possible to set the predetermined frequency such that one of those dielectric particles is caused to move to the first electric field zone A with the positive dielectrophoresis and the other dielectric particle is caused to move to the second electric field zone B with the negative dielectrophoresis. Thus, the alternate current voltage of the frequency in the range of higher than or equal to 100 kHz to lower than or equal to 400 kHz may be applied to separate (ii) the dielectric particle modified by the VHH antibody + the target substance and (iii) the solitary dielectric particle modified by the VHH antibody. Even when the crossover frequency has a certain band width, the predetermined frequency can be selected from the frequency range of higher than or equal to 150 kHz to lower than or equal to 350 kHz. To improve the separation performance, the predetermined frequency may be selected from the frequency range of higher than or equal to 200 kHz to lower than or equal to 300 kHz.

Thus, in separating two types of the particles 15 with the dielectrophoresis, the alternate current voltage of the predetermined frequency higher than a first frequency and lower than a second frequency is applied to the electrode set 1111. When the alternate current voltage of the first frequency is applied, both the positive dielectrophoresis and the negative dielectrophoresis act on one of the two types of the particles 15. When the alternate current voltage of the second frequency is applied, both the positive dielectrophoresis and the negative dielectrophoresis act on the other of the two types of the particles 15. In other words, the predetermined frequency is set to a value between the crossover frequency of the one type of the particles 15 and the crossover frequency of the other type of the particles 15.

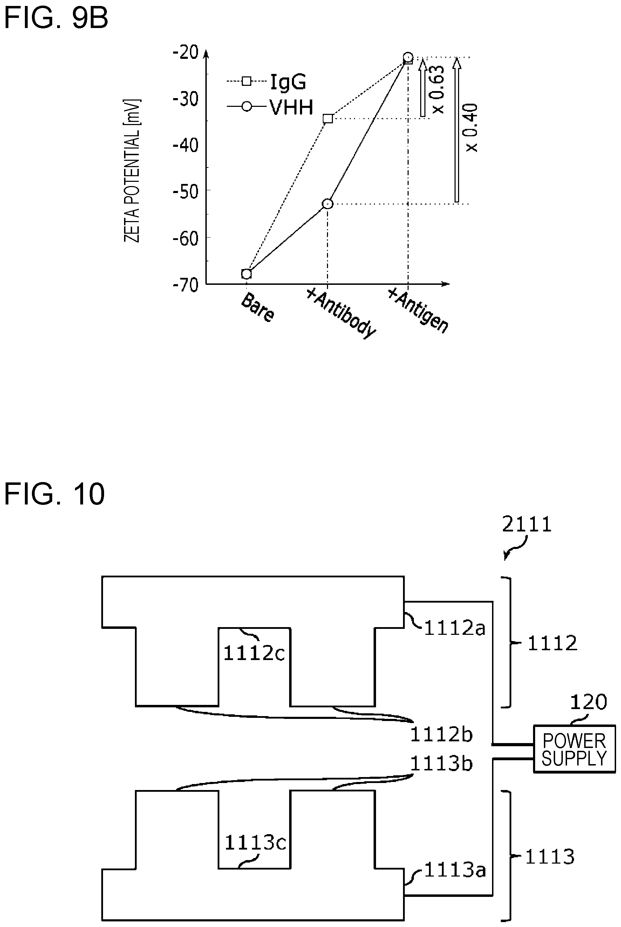

As described above, the frequency of the alternate current voltage applied to the electrode set 1111 is appropriately set in consideration of the crossover frequency. On that occasion, the crossover frequency is required to be different between the two or more types of the particles 15 to be separated. Here, attention is focused on zeta potentials of the particles 15 as the properties of the particles 15 affecting the crossover frequencies of the particles 15. Fig. 9A is a first graph depicting zeta potentials of particles in the embodiment and a comparative example. Fig. 9B is a second graph depicting the zeta potentials of the particles in the embodiment and the comparative example.

In Figs. 9A and 9B, the zeta potential of the dielectric particle modified by the VHH antibody in the embodiment and the zeta potential of the dielectric particle modified by the IgG antibody in the comparative example are indicated by a hollow circle and a hollow square, respectively. "Bare" denoted on the horizontal axis of each graph represents a dielectric particle alone, and "+Antibody" represents an antibody-modified dielectric particle alone in which the dielectric particle is modified by either one antibody, and "+Antigen" represents a complex particle in which the antibody-modified dielectric particle and a virus used as an antigen are bound to each other. Fig. 9A indicates the zeta potential of each type of the particle when the size of the dielectric particle is 1000 nanometers. Fig. 9B indicates the zeta potential of each type of the particle when the size of the dielectric particle is 300 nanometers.

The above-mentioned zeta potential is measured by the dielectrophoresis light scattering measurement method (so-called laser Doppler method). More specifically, movement of the dielectric particle is detected based on change in scattering intensity of a laser beam applied to the particle, and the zeta potential is calculated from mobility of the dielectric particle, the mobility being obtained from the detected result.

As depicted in Fig. 9A, in the example using the dielectric particle of 1000 nanometers, the dielectric particle modified by the VHH antibody in the embodiment and the dielectric particle modified by the IgG antibody exhibit the comparable zeta potentials (VHH: -26.4 mV. IgG: -24.1 mV) in the state of the complex particle. On the other hand, in the state of the antibody-modified dielectric particle alone, the dielectric particle modified by the VHH antibody in the embodiment and the dielectric particle modified by the IgG antibody exhibit the different zeta potentials (VHH: -44.2 V. IgG: -36.8 mV). Thus, with the binding of the virus, the zeta potential is changed in the dielectric particle modified by the VHH antibody to a greater extent than in the dielectric particle modified by the IgG antibody.

As depicted in the graph, by way of example, a ratio of the zeta potential of the complex particle to the zeta potential of the antibody-modified dielectric particle (i.e., the unbound particle) modified by the IgG antibody is 1 : 0.65. On the other hand, a ratio of the zeta potential of the complex particle to the zeta potential of the antibody-modified dielectric particle (i.e., the unbound particle) modified by the VHH antibody is 1 : 0.59.

As depicted in Fig. 9B, a similar tendency is also confirmed in the example using the dielectric particle of 300 nanometers. More specifically, the dielectric particle modified by the VHH antibody in the embodiment and the dielectric particle modified by the IgG antibody exhibit the comparable zeta potentials (VHH: -21.4 mV. IgG: -21.9 mV) in the state of the complex particle. On the other hand, in the state of the antibody-modified dielectric particle alone, the dielectric particle modified by the VHH antibody in the embodiment and the dielectric particle modified by the IgG antibody exhibit the different zeta potentials (VHH: -52.9 V. IgG: -34.6 mV). Thus, with the binding of the virus, the zeta potential is changed in the dielectric particle modified by the VHH antibody to a greater extent than in the dielectric particle modified by the IgG antibody.

As depicted in the graph, by way of example, a ratio of the zeta potential of the complex particle to the zeta potential of the antibody-modified dielectric particle (i.e., the unbound particle) modified by the IgG antibody is 1 : 0.63. On the other hand, a ratio of the zeta potential of the complex particle to the zeta potential of the antibody-modified dielectric particle (i.e., the unbound particle) modified by the VHH antibody is 1 : 0.40.

To generate a sufficient difference between the crossover frequencies to make the dielectrophoresis act on the two types of the particles in the different directions as described above, the ratio of the zeta potential of the complex particle to the zeta potential of the unbound particle is to be a value smaller than 0.65 and a value smaller than 0.63. The ratio of the zeta potential of the complex particle to the zeta potential of the unbound particle is to be a value greater than or equal to 0.40 and a value greater than or equal to 0.59. Thus, the ratio of the zeta potential of the complex particle to the zeta potential of the unbound particle is to be within the range greater than or equal to 0.40 and smaller than or equal to 0.62.

### [Advantageous Effects and so on]

As described above, the detection method according to the embodiment includes forming the complex particle 13 by binding the target substance 11 and the dielectric particle 12a modified by the single-domain antibody (for example, the VHH antibody) that is specifically bindable to the target substance 11, separating the complex particle 13 and the unbound particle 12 in the fluid, such as the solvent fluid of the sample 10, with the dielectrophoresis, the unbound particle 12 being the dielectric particle 12a not forming the complex particle 13, and detecting the target substance 11 contained in the separated complex particle 13 by using the imaging element 140.

In the detection method described above, the target substance 11 is captured as an antigen for a single-domain antibody, and the target substance 11 and the dielectric particle 12a form the complex particle 13 with the single-domain antibody interposed therebetween. The target substance 11 to which the dielectric particle 12a is bound is caused to move with the dielectrophoresis. On that occasion, the complex particle 13 can be selectively detected by separating the unbound particle 12 containing the dielectric particle 12a and being in the unbound state with the dielectrophoresis, and the target substance 11 correlated to the detected complex particle 13 can be detected.

Because a complex in which a magnetic particle and a fluorescent particle are bound to each other without interposition of a target substance and a complex in which the magnetic particle and the fluorescent particle are bound to each other with the target substance interposed therebetween exhibit the same behavior with application of a magnetic field, it is difficult to selectively detect the complex in which the magnetic particle and the fluorescent particle are bound to each other with the target substance interposed therebetween. Accordingly, that type of related-art detection method increases the influence of the false-positive detection and reduces the detection accuracy. By contrast, in the detection method according to the embodiment, because the unbound particle 12 not containing the target substance 11 and the complex particle 13 containing the target substance 11 exhibit the different behaviors, the complex particle 13 containing the target substance 11 can be selectively detected. As a result, the false-positive detection caused by the nonspecific adsorption can be reduced, and the detection accuracy of the target substance 11 can be improved.

For instance, in separating the complex particle 13 and the unbound particle 12, the alternate current voltage of the predetermined frequency may be applied such that one of the positive dielectrophoresis and the negative dielectrophoresis acts on the complex particle 13 and the other of the positive dielectrophoresis and the negative dielectrophoresis acts on the unbound particle 12.

The above-described application of the alternate current voltage of the predetermined frequency enables the positive dielectrophoresis to act on one of the complex particle 13 and the unbound particle 12 and the negative dielectrophoresis to act on the other. Accordingly, the complex particle 13 can be separated from the unbound particle 12, and the target substance 11 contained in the separated complex particle 13 can be detected. Since those two types of the particles are caused to move in opposite directions with the positive dielectrophoresis and the negative dielectrophoresis, the complex particle 13 and the unbound particle 12 can be separated from each other. As a result, it is possible to selectively detect the complex particle 13, to reduce the false-positive detection caused by the nonspecific adsorption, and to improve the detection accuracy of the target substance 11.

In an example, the predetermined frequency may be a value higher than the first frequency and lower than the second frequency. Both the positive dielectrophoresis and the negative dielectrophoresis may act on the complex particle 13 due to an electric field gradient generated when the alternate current voltage of the first frequency is used instead of the alternate current voltage of the predetermined frequency, and both the positive dielectrophoresis and the negative dielectrophoresis may act on the unbound particle 12 due to an electric field gradient generated when the alternate current voltage of the second frequency is used instead of the alternate current voltage of the predetermined frequency.

With the feature described above, the complex particle 13 and the unbound particle 12 can be separated from each other by applying the alternate current voltage of the predetermined frequency between the first frequency, namely the crossover frequency of the complex particle 13, and the second frequency, namely the crossover frequency of the unbound particle 12. Thus, the alternate current voltage of the appropriate frequency can be applied to be able to selectively detect the complex particle 13. As a result, the false-positive detection caused by the nonspecific adsorption can be reduced, and the detection accuracy of the target substance 11 can be improved.

In an example, the ratio of the zeta potential of the complex particle 13 to the zeta potential of the unbound particle 12 may be set to be smaller than or equal to 1. 0 or to be greater than or equal to 0.40 and smaller than or equal to 0.62.

With the feature described above, particle configurations enabling the complex particle 13 and the unbound particle 12 to be separated can be selected in consideration of the zeta potentials affecting the crossover frequencies of the complex particle 13 and the unbound particle 12. Particularly, whether an appropriate detection system can be constructed can be evaluated depending on whether the ratio of the zeta potential of the complex particle 13 to the zeta potential of the unbound particle 12 is smaller than or equal to 1. 0 and more preferably in the range of greater than or equal to 0.40 and smaller than or equal to 0.62. As a result, easiness in application of the detection method can be improved.

In an example, the dielectric particle 12a may contain a fluorescent substance, and detecting the target substance 11 may include illuminating the separated complex particle 13 with excitation light, taking an image of fluorescence generated from the fluorescent substance contained in the complex particle 13 with the imaging element 140, and detecting the target substance 11 contained in the complex particle 13.

With the feature described above, the dielectric particle 12a can be detected with high accuracy by utilizing reduction of the fluorescence emission. For instance, even when the particle size of the dielectric particle 12a cannot be increased due to limitations on the crossover frequency, the zeta potential, and so on, the dielectric particle 12a can be detected with high accuracy. In other words, with the improvement in detection sensitivity, a selection range of the dielectric particle 12a can be expanded, and the detection method according to the embodiment can be applied to a more variety of detection systems.

The detection device 100 according to the embodiment is the detection device 100 detecting the target substance 11 with the dielectrophoresis and including the dielectric particle 12a modified by the single-domain antibody (for example, the VHH antibody) that is specifically bindable to the target substance 11, the separator 110 separating the complex particle 13 formed by binding of the target substance 11 and the dielectric particle 12a from the unbound particle 12 in the fluid, such as the solvent fluid of the sample 10, with the dielectrophoresis, the unbound particle 12 being the dielectric particle 12a not forming the complex particle 13, and the imaging element 140 used to detect the target substance 11 contained in the separated complex particle 13.

The above-described detection device 100 can provide the detection device 100 implementing the above-described detection method.

In an example, the separator 110 may include the electrode set 1111 including the first electrode 1112 and the second electrode 1113 that are arranged apart from each other, the first electrode 1112 may include the first base portion 1112a extending in a predetermined direction and one or more first protruding portions 1112b protruding from the first base portion 1112a in a direction intersecting the predetermined direction in a convex form toward the second electrode 1113, and the second electrode 1113 may include the second base portion 1113a extending in the predetermined direction and one or more second protruding portions 1113b protruding from the second base portion 1113a in a direction intersecting the predetermined direction in a convex form toward the first electrode 1112. When the alternate current voltage is applied to the electrode set 1111, the first protruding portion 1112b and the second protruding portion 1113b may form the first electric field zone A including the position at which the distance between the first electrode 1112 and the second electrode 1113 is shortest and may form the second electric field zone B including the position at which the distance between the first electrode 1112 and the second electrode 1113 is longest, and the intensity of the electric field may be lower in the second electric field zone B than in the first electric field zone A.

With the feature described above, the intensity of the electric field to be formed can be given with a gradient depending on the shape of the electrode set 1111. Thus, since the nonuniform electric field can be generated in accordance with the design of the electrode set 1111, the electric field suitable for the detection system can be selectively used. As a result, the application range of the detection device 100 can be expanded.

The dielectric particle 12a in the embodiment is modified by the single-domain antibody that is specifically bindable to the target substance 11.

The dielectric particle 12a modified as mentioned above is useful in the detection device 100 or the detection method for detecting the target substance 11 with the dielectrophoresis.

### (Modifications)

While the detection device and the detection method according to one or more aspects of the present disclosure have been described with reference to the embodiments, the present disclosure is not limited to those embodiments. Other variously modified embodiments conceivable by those skilled in the art may also fall within the scope of the one or more aspects of the present disclosure without departing from the gist of the present disclosure.

While, in the above-described embodiment, the electrode set 1111 is arranged on the first substrate 111 as illustrated in Fig. 3, the shape and the arrangement of the electrode set are not limited to the illustrated ones. Fig. 10 is a first plan view illustrating a configuration of an electrode set according to a modification. As illustrated in Fig. 10, by way of example, an electrode set 2111 may be disposed on the first substrate 111. In the electrode set 2111 illustrated in Fig. 6, the first protruding portion 1112b of the first electrode 1112 and the second protruding portion 1113b of the second electrode 1113 face each other in the second direction (i.e., the up-down direction on the drawing sheet of Fig. 10). Even the electrode set 2111 described above can also generate the nonuniform electric field with the application of the alternate current voltage.

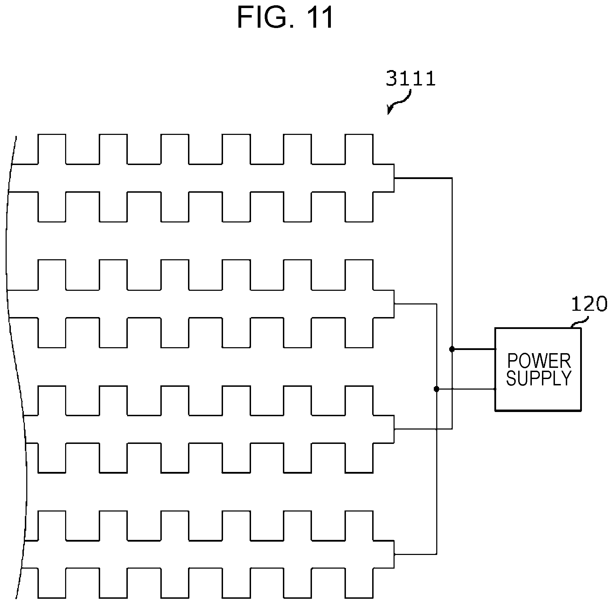

The number of electrodes included in the electrode set is not limited to two and may be three or more. Fig. 11 is a second plan view illustrating a configuration of an electrode set according to a modification. As illustrated in Fig. 11, by way of example, an electrode set 3111 may be disposed on the first substrate 111. The electrode set 3111 illustrated in Fig. 11 includes three or more electrodes, and a phase difference is given between the alternate current voltages applied to the adjacent electrodes. The electrode set 3111 is also called a Castellated electrode in some cases.

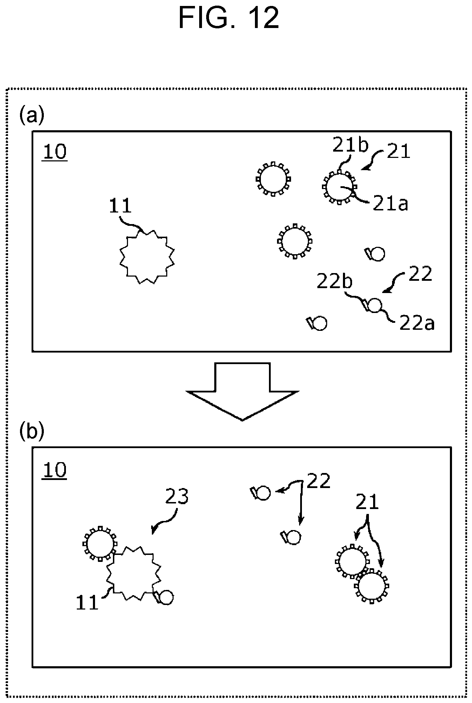

While, in the above-described embodiment, the complex particle 13 is illustrated in Fig. 5, the configuration of the complex particle is not limited to the illustrated one. Fig. 12 illustrates a formation process of a complex particle according to a modification. The above embodiment has been described, by way of example, in connection with the case in which the fluorescent substance is contained in the dielectric particle 12a, but a dielectric particle 21a and a fluorescent particle 22a may be separate particles as illustrated in Fig. 12.

As illustrated in (a) of Fig. 12, a sample 10 is produced by mixing the target substance 11, an unbound particle 21, and an antibody-modified fluorescent particle 22. The unbound particle 21 is made of the dielectric particle 21a of greater than or equal to 500 nanometers to smaller than or equal to 1000 nanometers which is modified by an antibody 21b of about 5 nanometers. The antibody-modified fluorescent particle 22 is made of the fluorescent particle 22a of about 300 nanometers modified by an antibody 22b of about 5 nanometers. Sizes of the above-mentioned particles and antibodies are not limited to the above-mentioned values.

A polystyrene particle can be used as the dielectric particle 21a, but the type of the dielectric particle 21a is not limited to the polystyrene particle. A VHH antibody can be used as each of the antibodies 21b and 22b, but those antibodies are not limited to the VHH antibody. The antibodies 21b and 22b may be different from each other.

When the sample 10 illustrated in Fig. 12(a) is left to stand for a predetermined time under a predetermined temperature, the target substance 11, the unbound particle 21, and the antibody-modified fluorescent particle 22 are bound to each other by the antigen-antibody reaction and a complex particle 23 is formed as illustrated in (b) of Fig. 12. On that occasion, a size of the complex particle 23 is greater than or equal to 900 nanometers and smaller than or equal to 1400 nanometers. The unbound particles 12 having not bound to the target substances 11 remain in an isolated or aggregated state.

Thus, by setting the dielectric particle 21a to be larger than the fluorescent particle 22a, it is possible to increase a difference between the size of the complex particle 23 and the size of the unbound particle 21 and to more reliably separate the complex particle 23 and the unbound particle 21 with the dielectrophoresis.

Because the frequency of the alternate current voltage to separate the particles 15 based in the zeta potential is changed depending on the size of the particles 15, the zeta potential may take a value outside the range of the zeta potential described in the above-described embodiment. The particles 15 for use in the detection of the target substance 11 may be determined by using the zeta potential as a parameter for selection of the particles 15 that can be separated with the detection device 100, and by measuring the crossover frequencies for the individual particles 15.

### Industrial Applicability

The present disclosure can be utilized as the detection device for detecting the target substance such as an influenza virus.

### Reference Signs List

10 sample
11 target substance
12, 21 unbound particle
12a, 21a dielectric particle
12b specifically binding substance
13, 23 complex particle
14 contaminant
15 particle
21b, 22b antibody
22 antibody-modified fluorescent particle
22a fluorescent particle
100 detection device
110 separator
111 first substrate
112 spacer
113 second substrate
120 power supply
130 light source
131 illumination light
132 detection light
140 imaging element
141 camera
150 detector
1111, 2111, 3111 electrode set
1112 first electrode
1112a first base portion
1112b first protruding portions
1112c first recessed portion
1113 second electrode
1113a second base portion
1113b second protruding portions
1113c second recessed portion
1121 space
1131 supply hole
1132 discharge hole

## Claims

1. A detection method comprising:
forming a complex by binding a target substance and a dielectric particle modified by a single-domain antibody that is bindable to the target substance;
separating the complex and an unbound particle in a fluid with dielectrophoresis, the unbound particle being the dielectric particle not forming the complex; and
detecting the target substance contained in the separated complex with an imaging element.

2. The detection method according to claim 1,
wherein, in separating the complex and the unbound particle, an alternate current voltage of a predetermined frequency is applied such that one of positive dielectrophoresis and negative dielectrophoresis acts on the complex and the other of the positive dielectrophoresis and the negative dielectrophoresis acts on the unbound particle.

3. The detection method according to claim 2,
wherein the predetermined frequency is a value higher than a first frequency and lower than a second frequency,
both the positive dielectrophoresis and the negative dielectrophoresis act on the complex due to an electric field gradient generated when the alternate current voltage of the first frequency instead is used instead of the alternate current voltage of the predetermined frequency, and
both the positive dielectrophoresis and the negative dielectrophoresis act on the unbound particle due to an electric field gradient generated when the alternate current voltage of the second frequency is used instead of the alternate current voltage of the predetermined frequency.

4. The detection method according to any one of claims 1 to 3,
wherein a ratio of a zeta potential of the complex to a zeta potential of the unbound particle is smaller than or equal to 1. 0.

5. The detection method according to any one of claims 1 to 4,
wherein the dielectric particle contains a fluorescent substance, and
detecting the target substance comprises illuminating the separated complex with excitation light, taking an image of fluorescence generated from the fluorescent substance contained in the complex with the imaging element, and detecting the target substance contained in the complex.

6. A detection device detecting a target substance with dielectrophoresis, comprising:
a dielectric particle modified by a single-domain antibody that is bindable to the target substance;
a separator separating a complex formed by binding of the target substance and the dielectric particle from an unbound particle in a fluid with the dielectrophoresis, the unbound particle being the dielectric particle not forming the complex; and
an imaging element used to detect the target substance contained in the separated complex.

7. The detection device according to claim 6,
wherein the separator includes an electrode set including a first electrode and a second electrode that are arranged apart from each other,
the first electrode includes a first base portion extending in a predetermined direction and one or more first protruding portions protruding from the first base portion in a direction intersecting the predetermined direction in a convex form toward the second electrode,
the second electrode includes a second base portion extending in the predetermined direction and one or more second protruding portions protruding from the second base portion in a direction intersecting the predetermined direction in a convex form toward the first electrode,
when an alternate current voltage is applied to the electrode set, the first protruding portion and the second protruding portion form a first electric field zone including a position at which a distance between the first electrode and the second electrode is shortest and form a second electric field zone including a position at which the distance between the first electrode and the second electrode is longest, and
intensity of an electric field is lower in the second electric field zone than in the first electric field zone.

8. A dielectric particle modified by a single-domain antibody that is bindable to a target substance.

9. A detection method comprising:
separating a complex and an unbound particle in a fluid with dielectrophoresis, and
detecting a target substance contained in the separated complex with an imaging element,
wherein the complex is formed by binding of the target substance and a dielectric particle modified by a single-domain antibody that is bindable to the target substance, and
the unbound particle is the dielectric particle not forming the complex.

10. A detection method comprising:
mixing target substances and dielectric particles modified by single-domain antibodies, thereby producing a fluid containing complexes in which first target substances and first dielectric particles are bound to each other and second dielectric particles not bound to the target substances, each of the dielectric particles being modified by one or more of the single-domain antibodies;
generating an alternate current voltage between a first electrode in a shape including protruding and recessed portions and a second electrode in a shape including protruding and recessed portions, thereby generating a nonuniform electric field between the first electrode and the second electrode, the nonuniform electric field passing through the mixture fluid separating the first complexes and the second dielectric particles; and
detecting the separated first complexes with an imaging element,
the target substances including the first target substances,
the dielectric particles including the first dielectric particles and the second dielectric particles.
